# FASCICULE DE BREVET EUROPEEN

(11) **EP 0 853 471 B1**
(45) Date de publication et mention de la délivrance du brevet: **31.10.2001**
(21) Numéro de dépôt: 96932627.1
(22) Date de dépôt: 20.09.1996
(51) Int. Cl.: A61K 7/13

(54) **COMPOSITION DE TEINTURE D'OXYDATION DES FIBRES KERATINIQUES ET PROCEDE DE TEINTURE METTANT EN OEUVRE CETTE COMPOSITION**
MITTEL ZUM OXIDATIVEN FÄRBEN VON KERATINFASERN UND FÄRBEVERFAHREN UNTER DESSEN VERWENDUNG
COMPOSITION FOR OXIDATION DYEING KERATIN FIBRES, AND DYEING METHOD USING SAME

(30) Priorité: 25.09.1995 FR 9511224
(43) Date de publication de la demande: 22.07.1998
(73) Titulaire: L'OREAL, 75008 Paris (FR)
(72) Inventeur: AUDOUSSET, Marie-Pascale, F-92600 Asnières (FR)
(74) Mandataire: Miszputen, Laurent
(86) Numéro de dépôt international: FR9601473
(87) Numéro de publication internationale: WO9711674

(56) Documents cités:
- EP-A- 0 428 441
- EP-A- 0 465 339
- EP-A- 0 465 340
- EP-A- 0 722 710
- DE-A- 3 031 709

## Description

La présente invention a pour objet une composition pour la teinture d'oxydation des fibres kératiniques, en particulier des fibres kératiniques humaines telles que les cheveux, comprenant au moins une base d'oxydation convenablement sélectionnée, au moins un méta-aminophénol à titre de premier coupleur et de la 4-hydroxyindoline et/ou au moins l'un de ses sels d'addition avec un acide à titre de deuxième coupleur, ainsi que le procédé de teinture mettant en oeuvre cette composition avec un agent oxydant.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés indoliques ou indoliniques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Pour la réalisation de nuances naturelles ou à reflets, il est courant d'utiliser des compositions tinctoriales comprenant un dérivé de paraphénylènediamine à titre de base d'oxydation et un dérivé de méta-aminophénol à titre de coupleur. Les colorations obtenues avec de telles compositions tinctoriales ne sont cependant pas entièrement satisfaisantes car elles présentent généralement une résistance médiocre à la lumière.

D'autre part, il a déjà été proposé, notamment dans la demande de brevet FR 2 008 797, des compositions pour la teinture d'oxydation en milieu alcalin des fibres kératiniques comprenant au moins une base d'oxydation telle que par exemple la paraphénylènediamine ou un dérivé de paraphénylènediamine, en association avec un coupleur indolinique comme par exemple la 4-hydroxyindoline. Ces compositions ne sont pas non plus entièrement satisfaisantes car elles conduisent à des colorations présentant également une résistance médiocre à la lumière.

Or, la demanderesse vient maintenant de découvrir qu'il est possible d'obtenir de nouvelles teintures en milieu acide, neutre ou alcalin, capables d'engendrer des colorations puissantes, et résistant bien aux diverses agressions que peuvent subir les cheveux et en particulier à l'action de la lumière, en associant au moins une base d'oxydation convenablement sélectionnée, au moins un méta-aminophénol à titre de premier coupleur et de la 4-hydroxyindoline et/ou au moins l'un de ses sels d'addition avec un acide à titre de second coupleur. Ce résultat est d'autant plus inattendu et surprenant que, comme indiqué précédemment, les deux coupleurs, lorsqu'ils sont utilisés séparément en association avec une même base d'oxydation conforme à l'invention, conduisent à des colorations sur cheveux qui sont peu résistantes à la lumière.

Cette découverte est à la base de la présente invention.

L'invention a donc pour premier objet une composition pour la teinture d'oxydation des fibres kératiniques et en particulier des fibres kératiniques humaines telles que les cheveux, caractérisée par le fait qu'elle comprend, dans un milieu approprié pour la teinture :
- au moins une base d'oxydation choisie parmi les paraphénylènediamines et/ou les bis-phénylalkylènediamines,
- au moins un méta-aminophénol à titre de premier coupleur,
- au moins un second coupleur choisi parmi la 4-hydroxyindoline et ses sels d'addition avec un acide.

La composition de teinture d'oxydation conforme à l'invention permet d'obtenir des colorations puissantes aux nuances variées, peu sélectives et présentant d'excellentes propriétés de résistance à la fois vis à vis des agents atmosphériques tels que la lumière et les intempéries et vis à vis de la transpiration et des différents traitements que peuvent subir les cheveux (shampooings, déformations permanentes). Ces propriétés sont particulièrement remarquables vis à vis de la lumière.

L'invention a également pour objet un procédé de teinture d'oxydation des fibres kératiniques mettant en oeuvre cette composition.

Parmi les méta-aminophénols utilisables à titre de premier coupleur dans les compositions conformes à l'invention, on peut citer les composés répondant à la formule (I) suivante, et leurs sels d'addition avec un acide : 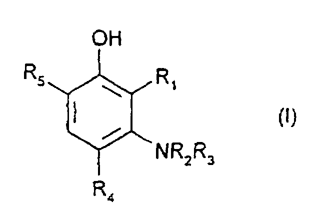 dans laquelle :
R₁ et R₄, identiques ou différents, représentent un atome d'hydrogène ou d'halogène, un radical alkyle en C₁-C₄ ou alcoxy en C₁-C₄,
R₂ représente un atome d'hydrogène, un radical alkyle en C₁-C₄, monohydroxyalkyle en C₁-C₄, polyhydroxyalkyle en C₂-C₄, trifluoroalkyle en C₁-C₄ ou carbamoylméthyle,
R₃ représente un atome d'hydrogène, un radical alkyle en C₁-C₄ ou bien encore forme, avec R₂ et l'atome d'azote, un hétérocycle à 5 ou 6 sommets,
R₅ représente un atome d'hydrogène ou d'halogène, un radical alkyle en C₁-C₄, alcoxy en C₁-C₄, monohydroxyalkyle en C₁-C₄ ou polyhydroxyalkyle en C₂-C₄.

Parmi les méta-aminophénols de formule (I) ci-dessus, utilisés à titre de premier coupleur dans les compositions tinctoriales conformes à l'invention, on peut plus particulièrement citer le 3-amino phénol, le 5-amino 2-méthoxy phénol, le 5-amino 4-chloro 2-méthyl phénol, le 5-amino 2,4-diméthoxy phénol, le 5-amino 2-β-hydroxyéthyloxy phénol, le 5-N-(β-hydroxyéthyl)amino 4-méthoxy 2-méthyl phénol, le 5-amino 4-méthoxy 2-méthyl phénol, le 5-N-(γ-hydroxypropyl)amino 2-méthyl phénol, le 5-N-(β-hydroxyéthyl)amino 2-méthyl phénol, le 5-amino 2-méthyl phénol, le 3-N-(carbamoylméthyl)amino phénol, le 5-N-(carbamoylméthyl)amino 2-méthyl phénol, le 3-N,N-(diméthyl)amino phénol, le 3-N,N-(diéthyl)amine phénol, le 3-amino 2,4-dichloro phénol, le 3-amino 4,6-dichloro phénol, le 5-amino 6-chloro 2-méthyl phénol, le 2-chloro 5-N-(2',2',2'-trifluoroéthyl)amino phénol, le 5-amino 4-chloro 2-méthyl phénol, le 3-N-(cyclopentyl)amino phénol, et leurs sels d'addition avec un acide.

Parmi les paraphénylènediamines utilisables à titre de base d'oxydation dans les compositions conformes à l'invention, on peut citer les composés répondant à la formule (II) suivante, et leurs sels d'addition avec un acide : 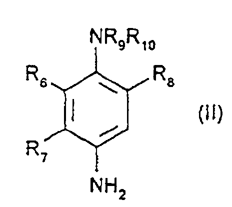 dans laquelle :
R₆, R₇ et R₈, identiques ou différents, représentent un atome d'hydrogène ou d'halogène, un radical alkyle en C₁-C₄, alcoxy en C₁-C₄, sulfo, carboxy, monohydroxyalkyle en C₁-C₄ ou polyhydroxyalkyle en C₂-C₄,
R₉ et R₁₀, identiques ou différents, représentent un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), carbamylalkyle en C₁-C₄, mésylaminoalkyle en C₁-C₄, acétylaminoalkyle en C₁-C₄, uréidoalkyle en C₁-C₄, carbalcoxy(C₁-C₄)aminoalkyle(C₁-C₄), sulfoalkyle en C₁-C₄, pipéridinoalkyle en C₁-C₄, morpholinoalkyle en C₁-C₄, phényle ou phényle substitué en position para par un groupement amino, ou bien encore R₉ et R₁₀ forment, conjointement avec l'atome d'azote auquel ils sont liés, un hétérocycle pipéridino ou morpholino;
étant entendu que si R₉ et R₁₀ ne représentent pas simultanément un atome d'hydrogène, alors un au moins des radicaux R₆, R₈ doit représenter un atome d'hydrogène.

Parmi les radicaux alkyle en C₁-C₄ et alcoxy en C₁-C₄ des formules (I) et (II) ci-dessus, on peut citer notamment les radicaux méthyle, éthyle, propyle, méthyloxy et éthyloxy.

Parmi les paraphénylènediamines de formule (II) ci-dessus, utilisées à titre de base d'oxydation dans les compositions tinctoriales conformes à l'invention, on peut plus particulièrement citer la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylenediamine, la 2-méthyl 5-méthoxy paraphénylènediamine, la 2,6-diméthyl 5-méthoxy paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-amino N,N-bis-(β-hydroxyéthyl) 3-méthyl aniline, la 4-amino 3-chloro N,N-bis-(β-hydroxyéthyl) aniline, la 4-amino N,N-(éthyl, carbamylméthyl) aniline, la 4-amino 3-méthyl N,N-(éthyl, carbamylméthyl) aniline, la 4-amino N,N-(éthyl, β-pipéridinoéthyl) aniline, la 4-amino 3-méthyl N,N-(éthyl, β-pipéridinoéthyl) aniline, la 4-amino N,N-(éthyl, β-morpholinoéthyl) aniline, la 4-amino 3-méthyl N,N-(éthyl, β-morpholinoéthyl) aniline, la 4-amino N,N-(éthyl, β-acétylaminoéthyl) aniline, la 4-amino N-(β-méthoxyéthyl) aniline, la 4-amino 3-méthyl N,N-(éthyl, β-acétylaminoéthyl) aniline, la 4-amino N,N-(éthyl, β-mésylaminoéthyl) aniline, la 4-amino 3-méthyl N,N-(éthyl, β-mésylaminoéthyl) aniline, la 4-amino N,N-(éthyl, β-sulfoéthyl) aniline, la 4-amino 3-méthyl N,N-(éthyl, β-sulfoéthyl) aniline, la N-[(4'-amino)phényl] morpholine, la N-[(4'-amino)phényl] pipéridine, la 2-β-hydroxyéthyl paraphénylènediamine, la 2-fluoro paraphénylènediamine, la 2-carboxy paraphénylènediamine, la 2-sulfo paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl para-phénylènediamine, la 2-β-hydroxyéthyloxy paraphénylènediamine, et leurs sels d'addition avec un acide.

Parmi les paraphénylènediamines de formule (II) ci-dessus, utilisées à titre de base d'oxydation dans les compositions tinctoriales conformes à l'invention, sont plus particulièrement préférées la paraphénylènediamine, la paratoluylènediamine, la 2,6-diméthyl paraphénylènediamine, la 2-β-hydroxyéthyl paraphénylènediamine, la 2-isopropyl paraphénylènediamine, la N-(β-hydroxypropyl) paraphénylènediamine, la N,N-bis-(β-hydroxyéthyl) paraphénylènediamine, la 4-amino N-(β-méthoxyéthyl) aniline, et leurs sels d'addition avec un acide.

Parmi les bis-phénylalkylènediamines utilisables à titre de base d'oxydation dans les compositions tinctoriales conformes à l'invention, on peut plus particulièrement citer les composés répondant à la formule (III) suivante, et leurs sels d'addition avec un acide: 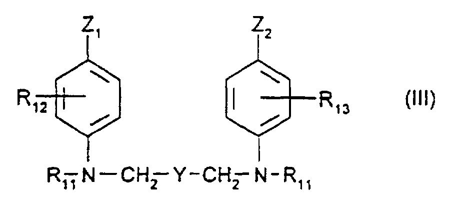 dans laquelle :
Z₁ et Z₂, identiques ou différents, représentent un radical hydroxyle ou NHR₁₄ dans lequel R₁₄ représente un atome d'hydrogène ou un radical alkyle en C₁-C₄,
R₁₁ représente un atome d'hydrogène, un radical alkyle en C₁-C₄, monohydroxyalkyle en C₁-C₄, polyhydroxyalkyle en C₂-C₄ ou aminoalkyle en C₁-C₄ dont le reste amino peut être substitué,
R₁₂ et R₁₃, identiques ou différents, représentent un atome d'hydrogène ou d'halogène ou un radical alkyle en C₁-C₄,
Y représente un radical pris dans le groupe constitué par les radicaux suivants : 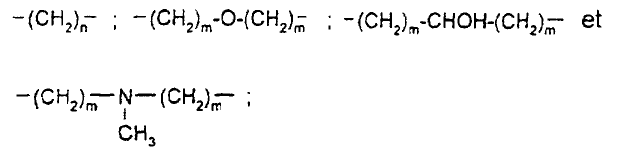
dans lesquels n est un nombre entier compris entre 0 et 8 inclusivement et m est un nombre entier compris entre 0 et 4 inclusivement.

Parmi les radicaux alkyle en C₁-C₄ de la formule (III) ci-dessus, on peut citer notamment les radicaux méthyle, éthyle et propyle.

Parmi les bis-phénylalkylènediamines de formules (III) ci-dessus, utilisées à titre de base d'oxydation dans les compositions tinctoriales conformes à l'invention, on peut plus particulièrement citer le N,N'-bis-(β-hydroxyéthyl) N,N'-bis-(4'-amino-phényl) 1,3-diamino propanol, la N,N'-bis-(β-hydroxyéthyl) N,N'-bis-(4'-amino-phényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

Parmi ces bis-phénylalkylènediamines de formule (III), le N,N'-bis-(β-hydroxyéthyl) N,N'-bis-(4'-aminophényl) 1,3-diamino propanol est particulièrement préféré.

Les sels d'addition avec un acide utilisables dans le cadre des compositions tinctoriales de l'invention sont notamment choisis parmi les chlorhydrates, les bromhydrates, les sulfates et les tartrates.

La ou les bases d'oxydation conformes à l'invention, c'est à dire la ou les paraphénylènediamines et/ou la ou les bis-phénylalkylènediamines, représentent de préférence de 0,0005 à 12 % en poids environ du poids total de la composition tinctoriale, et encore plus préférentiellement de 0,005 à 6 % en poids environ de ce poids.

Le ou les méta-aminophénols de formule (I), utilisés à titre de premier coupleur dans les compositions tinctoriales conformes à l'invention, représentent de préférence de 0,0001 à 10 % en poids environ du poids total de la composition tinctoriale et encore plus préférentiellement de 0,005 à 5 % en poids environ de ce poids.

La 4-hydroxyindoline et/ou son ou ses sels d'addition avec un acide, utilisés à titre de second coupleur dans les compositions tinctoriales conformes à l'invention, représentent de préférence de 0,0001 à 10 % en poids environ du poids total de la composition tinctoriale et encore plus préférentiellement de 0,005 à 5 % en poids environ de ce poids.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (IV) suivante : 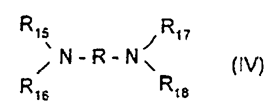 dans laquelle R est un reste propylène éventuellement substitué par un groupement hydroxyle ou un radical alkyle en C₁-C₄ ; R₁₅, R₁₆, R₁₇ et R₁₈, identiques ou différents, représentent un atome d'hydrogène, un radical alkyle en C₁-C₄ ou hydroxyalkyle en C₁-C₄.

La composition tinctoriale conforme à l'invention peut encore contenir, en plus des colorants définis ci-dessus, d'autres bases d'oxydation et/ou d'autres coupleurs et/ou des colorants directs, notamment pour modifier les nuances ou les enrichir en reflets.

La composition tinctoriale selon l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir ce ou ces éventuels composés complémentaires de manière telle que les propriétés avantageuses attachées intrinsèquement à l'association ternaire conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLES

On a réalisé les compositions tinctoriales 1 à 3 suivantes:

| **COMPOSITION** | **1(*)** | **2(*)** | **3 (**)** |
|---|---|---|---|
| Paraphénylènediamine (en moles) | 3.10⁻³ | 3.10⁻³ | 3.10⁻³ |
| 3-amino phénol (en moles) | 3.10⁻³ | - | 1,5.10⁻³ |
| 4-hydroxyindoline (en moles) | - | 3.10⁻³ | 1,5.10⁻³ |
| Support de teinture commun | (***) | (***) | (***) |
| Eau déminéralisée q.s.p. | 100 g | 100 g | 100 g |

| | | | |
|---|---|---|---|
| (*) : composition tinctoriale ne faisant pas partie de l'invention | | | |
| (**) : composition tinctoriale conforme à l'invention | | | |
| (***) : Support de teinture commun : - Alcool oléique polyglycérolé à 2 moles de glycérol 4,0 g - Alcool oléique polyglycérolé à 4 moles de glycérol, à 78 % de matières actives (M.A.) 5,69 g M.A. - Acide oléique 3,0 g - Amine oléique à 2 moles d'oxyde d'éthylène vendue sous la dénomination commerciale ETHOMEEN O12   par la société AKZO 7,0 g - Laurylamino succinamate de diéthylaminopropyle, sel de sodium, à 55 % de M.A. 3,0 g M.A. - Alcool oléique 5,0 g - Diéthanolamide d'acide oléique 12,0 g - Propylèneglycol 3,5 g - Alcool éthylique 7,0 g - Dipropylèneglycol 0,5 g - Monométhyléther de propylèneglycol 9,0 g - Métabisulfite de sodium en solution aqueuse, à 35 % de M.A. 0,455 g M.A. - Acétate d'ammonium 0,8 g - Antioxydant, séquestrant q.s. - Parfum, conservateur q.s. - Ammoniaque à 20 % de NH₃ 10,0 g | | | |

Au moment de l'emploi, on a mélangé chaque composition tinctoriale 1 à 3 avec une quantité égale en poids d'une solution d'eau oxygénée à 20 volumes (6 % en poids).

Chaque mélange résultant a été appliqué pendant 30 minutes sur des mèches de cheveux gris naturels à 90 % de blancs. Les mèches de cheveux ont ensuite été rincées, lavées avec un shampooing standard puis séchées.

La couleur des mèches a ensuite été évaluée dans le système MUNSELL au moyen d'un colorimètre CM 2002 MINOLTA.

Les mèches de cheveux ainsi teintes ont ensuite subi un test de résistance à la lumière. L'appareil utilisé pour réaliser le test de résistance à la lumière était un SUNTEST HANAU, équipé d'une lampe xénon permettant le passage des rayons UV dans une gamme de longueur d'ondes comprise entre 300 et 830 nm.

Les mèches ont été fixées sur un support en carton qui a été introduit dans l'appareil et irradié pendant 18 heures, ce qui correspond à une exposition à la lumière naturelle d'environ trois semaines.

La lampe xénon a été refroidie, pendant le test, au moyen d'un flux d'air d'environ 60 m³ / heure. Un second flux d'air d'environ 60 m³ / heure également refroidissait les mèches.
La température au niveau des mèches était d'environ 45°C.
L'intensité de la lumière au niveau des mèches étaient d'environ 150 klx.
L'intensité de l'irradiation était d'environ 830 W/m².

La couleur des mèches après le test a de nouveau été évaluée dans le système MUNSELL au moyen d'un colorimètre CM 2002 MINOLTA.

La différence de couleur de chaque mèche avant et après le test de résistance à la lumière reflète la dégradation de la coloration due à l'action de la lumière et a été calculée en appliquant la formule de NICKERSON :
**ΔE = 0,4 CoΔH + 6ΔV + 3 ΔC**, telle que décrite par exemple dans "Couleur, Industrie et Technique" ; pages 14-17 ; vol. n° 5; 1978.

Dans cette formule, Δ**E** représente la différence de couleur entre deux mèches, Δ**H**, Δ**V** et Δ**C** représentent la variation en valeur absolue des paramètres **H**, **V** et **C**, et **Co** représente la pureté de la mèche par rapport à laquelle on désire évaluer la différence de couleur (pureté de la mèche avant le test).

Les résultats sont donnés dans le tableau Il ci-dessous :

| **EXEMPLE COMPOSITION)** | **Couleur avant le test** | **Couleur après le test** | **Dégradation de la couleur** | | | |
|---|---|---|---|---|---|---|
| | | | ΔH | ΔV | ΔC | ΔE |
| **1 (1)** | 10 RP 2,4 / 1,4 | 5,2 R 2,7 / 1,2 | 5,2 | 0,3 | 0,2 | 5,3 |
| **2 (2)** | 4,2 YR 2,7 / 1,4 | 5,6 YR 3,3 / 1,5 | 1,4 | 0,6 | 0,1 | 4,7 |
| **3 (3)** | 6,9 R 2,7 / 1,4 | 0,4 YR 2,8 / 1,3 | 3,5 | 0,1 | 0,1 | 2,9 |

Ces résultats montrent clairement que la composition tinctoriale de l'exemple 3 conforme à l'invention, c'est à dire comprenant une base d'oxydation (paraphénylènediamine), en association avec du méta-aminophénol à titre de premier coupleur et de la 4-hydroxyindoline à titre de second coupleur, conduit à une coloration résistant nettement mieux à la lumière que les colorations obtenues avec les compositions tinctoriales comparatives des exemples 1 et 2 (qui ne font pas partie de l'invention, car elles ne contiennent qu'un seul des deux coupleurs).

## Revendications

1. Composition pour la teinture d'oxydation des fibres kératiniques et en particulier des fibres kératiniques humaines telles que les cheveux, **caractérisée par le fait qu'**elle comprend, dans un milieu approprié pour la teinture :
- au moins une base d'oxydation choisie parmi les paraphénylènediamines et/ou les bis-phénylalkylènediamines,
- au moins un méta-aminophénol à titre de premier coupleur,
- au moins un second coupleur choisi parmi la 4-hydroxyindoline et ses sels d'addition avec un acide.

2. Composition selon la revendication 1, **caractérisée par le fait que** le (ou les) méta-aminophénol(s) utilisé(s) à titre de premier coupleur est (sont) choisi(s) parmi les composés répondant à la formule (I) suivante, et leurs sels d'addition avec un acide : 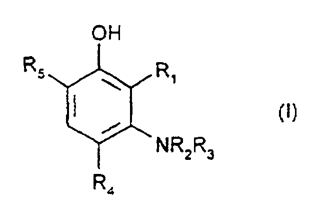 dans laquelle :
R₁ et R₄, identiques ou différents, représentent un atome d'hydrogène ou d'halogène, un radical alkyle en C₁-C₄ ou alcoxy en C₁-C₄,
R₂ représente un atome d'hydrogène, un radical alkyle en C₁-C₄, monohydroxyalkyle en C₁-C₄, polyhydroxyalkyle en C₂-C₄, trifluoroalkyle en C₁-C₄ ou carbamoylméthyle,
R₃ représente un atome d'hydrogène, un radical alkyle en C₁-C₄ ou bien encore forme, avec R₂ et l'atome d'azote, un hétérocycle à 5 ou 6 sommets,
R₅ représente un atome d'hydrogène ou d'halogène, un radical alkyle en C₁-C₄, alcoxy en C₁-C₄, monohydroxyalkyle en C₁-C₄ ou polyhydroxyalkyle en C₂-C₄.

3. Composition selon la revendication 2, **caractérisée par le fait que** le (ou les) méta-aminophénol(s) utilisé(s) à titre de premier coupleur est (sont) choisi(s) parmi le 3-amino phénol, le 5-amino 2-méthoxy phénol, le 5-amino 4-chloro 2-méthyl phénol, le 5-amino 2,4-diméthoxy phénol, le 5-amino 2-β-hydroxyéthyloxy phénol, le 5-N-(β-hydroxyéthyl)amino 4-méthoxy 2-méthyl phénol, le 5-amino 4-méthoxy 2-méthyl phénol, le 5-N-(γ-hydroxypropyl)amino 2-méthyl phénol, le 5-N-(β-hydroxyéthyl)amino 2-méthyl phénol, le 5-amino 2-méthyl phénol, le 3-N-(carbamoylméthyl)amino phénol, le 5-N-(carbamoylméthyl)amino 2-méthyl phénol, le 3-N,N-(diméthyl)amino phénol, le 3-N,N-(diéthyl)amino phénol, le 3-amino 2,4-dichloro phénol, le 3-amino 4,6-dichloro phénol, le 5-amino 6-chloro 2-méthyl phénol, le 2-chloro 5-N-(2',2',2'-trifluoroéthyl)amino phénol, le 5-amino 4-chloro 2-méthyl phénol, le 3-N-(cyclopentyl)amino phénol, et leurs sels d'addition avec un acide.

4. Composition selon l'une quelconque des revendications précédentes, **caractérisée par le fait que** les paraphénylènediamines sont choisies parmi les composés de formule (II) suivante, et leurs sels d'addition avec un acide : 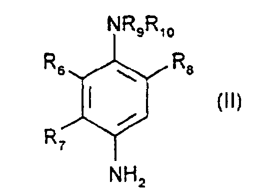 dans laquelle :
R₆, R₇ et R₈, identiques ou différents, représentent un atome d'hydrogène ou d'halogène, un radical alkyle en C₁-C₄, alcoxy en C₁-C₄, sulfo, carboxy, monohydroxyalkyle en C₁-C₄ ou polyhydroxyalkyle en C₂-C₄,
R₉ et R₁₀, identiques ou différents, représentent un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), carbamylalkyle en C₁-C₄, mésylaminoalkyle en C₁-C₄, acétylaminoalkyle en C₁-C₄, uréidoalkyle en C₁-C₄, carbalcoxy(C₁-C₄)aminoalkyle(C₁-C₄), sulfoalkyle en C₁-C₄, pipéridinoalkyle en C₁-C₄, morpholinoalkyle en C₁-C₄, phényle ou phényle substitué en position para par un groupement amino, ou bien encore R₉ et R₁₀ forment, conjointement avec l'atome d'azote auquel ils sont liés, un hélérocycle pipéridino ou morpholino ;
étant entendu que si R₉ et R₁₀ ne représentent pas simultanément un atome d'hydrogène, alors un au moins des radicaux R₆, R₈ doit représenter un atome d'hydrogène.

5. Composition selon la revendication 4, **caractérisée par le fait que** les paraphénylènediamines de formule (Il) sont choisies parmi la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la 2-méthyl 5-méthoxy paraphénylènediamine, la 2,6-diméthyl 5-méthoxy paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-amino N,N-bis-(β-hydroxyéthyl) 3-méthyl aniline, la 4-amino 3-chloro N,N-bis-(β-hydroxyéthyl) aniline, la 4-amino N,N-(éthyl, carbamylméthyl) aniline, la 4-amino 3-méthyl N,N-(éthyl, carbamylméthyl) aniline, la 4-amino N,N-(éthyl, β-pipéridinoéthyl) aniline, la 4-amino 3-méthyl N,N-(éthyl, β-pipéridinoéthyl) aniline, la 4-amino N,N-(éthyl, β-morpholinoéthyl) aniline, la 4-amino 3-méthyl N,N-(éthyl, β-morpholinoéthyl) aniline, la 4-amino N,N-(éthyl, β-acétylaminoéthyl) aniline, la 4-amino N-(β-méthoxyéthyl) aniline, la 4-amino 3-méthyl N,N-(éthyl, β-acétylaminoéthyl) aniline, la 4-amino N,N-(éthyl, β-mésylaminoéthyl) aniline, la 4-amino 3-méthyl N,N-(éthyl, β-mésylaminoéthyl) aniline, la 4-amino N,N-(éthyl, β-sulfoéthyl) aniline, la 4-amino 3-méthyl N,N-(éthyl, β-sulfoéthyl) aniline, la N-[(4'-amino)phényl] morpholine, la N-[(4'-amino)phényl] pipéridine, la 2-β-hydroxyéthyl paraphénylènediamine, la 2-fluoro paraphénylènediamine, la 2-carboxy paraphénylènediamine, la 2-sulfo paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, et leurs sels d'addition avec un acide.

6. Composition selon la revendication 5, **caractérisée par le fait que** les paraphénylènediamines de formule (II) sont choisies parmi la paraphénylènediamine, la paratoluylènediamine, la 2,6-diméthyl paraphénylènediamine, la 2-β-hydroxyéthyl paraphénylènediamine, la 2-isopropyl paraphénylènediamine, la N-(β-hydroxypropyl) paraphénylènediamine, la N,N-bis-(β-hydroxyéthyl) paraphénylènediamine, la 4-amino N-(β-méthoxyéthyl) aniline, et leurs sels d'addition avec un acide.

7. Composition selon l'une quelconque des revendications précédentes, **caractérisée par le fait que** les bis-phénylalkylènediamines sont choisies parmi les composés de formule (III) suivante, et leurs sels d'addition avec un acide : 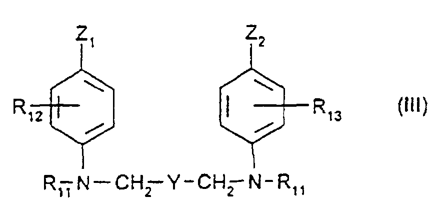 dans laquelle :
Z₁ et Z₂, identiques ou différents, représentent un radical hydroxyle ou NHR₁₄ dans lequel R₁₄ représente un atome d'hydrogène ou un radical alkyle en C₁-C₄, R₁₁ représente un atome d'hydrogène, un radical alkyle en C₁-C₄, monohydroxyalkyle en C₁-C₄, polyhydroxyalkyle en C₂-C₄ ou aminoalkyle en C₁-C₄ dont le reste amino peut être substitué,
R₁₂ et R₁₃, identiques ou différents, représentent un atome d'hydrogène ou d'halogène ou un radical alkyle en C₁-C₄,
Y représente un radical pris dans le groupe constitué par les radicaux suivants : 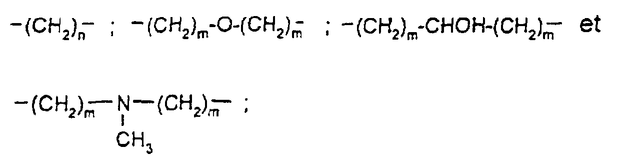 dans lesquels n est un nombre entier compris entre 0 et 8 inclusivement et m est un nombre entier compris entre 0 et 4 inclusivement.

8. Composition selon la revendication 7, **caractérisée par le fait que** les bis-phénylalkylènediamines de formules (III) sont choisies parmi le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

9. Composition selon l'une quelconque des revendications précédentes, **caractérisée par le fait que** les sels d'addition avec un acide sont choisis parmi les chlorhydrates, les bromhydrates, les sulfates et les tartrates.

10. Composition selon Tune quelconque des revendications précédentes, **caractérisée par le fait que** la ou les paraphénylènediamines et/ou la ou les bis-phénylalkylènediamines représentent de 0,0005 à 12 % en poids du poids total de la composition tinctoriale.

11. Composition selon la revendication 10, **caractérisée par le fait que** la ou les paraphénylènediamines et/ou la ou les bis-phénylalkylènediamines représentent de 0,005 à 6 % en poids du poids total de la composition tinctoriale.

12. Composition selon l'une quelconque des revendications précédentes, **caractérisée par le fait que** le ou les méta-aminophénols de formule (I) représentent de 0,0001 à 10 % en poids du poids total de la composition tinctoriale.

13. Composition selon la revendication 12, **caractérisée par le fait que** le ou les méta-aminophénols de formule (I) représentent de 0,005 à 5 % en poids du poids total de la composition tinctoriale.

14. Composition selon l'une quelconque des revendications précédentes, **caractérisée par le fait que** la 4-hydroxyindoline et/ou son ou ses sels d'addition avec un acide représentent de 0,0001 à 10 % en poids du poids total de la composition tinctoriale.

15. Composition selon la revendication 14, **caractérisée par le fait que** la 4-hydroxyindoline et/ou son ou ses sels d'addition avec un acide représentent de 0,005 à 5 % en poids du poids total de la composition tinctoriale.

16. Composition selon l'une quelconque des revendications précédentes, **caractérisée par le fait que** le milieu approprié pour la teinture (ou support) est constitué par de l'eau ou par un mélange d'eau et d'au moins un solvant organique choisi parmi les alcanols inférieurs en C₁-C₄, le glycérol, les glycols et éthers de glycols, les alcools aromatiques et leurs mélanges.

17. Composition selon l'une quelconque des revendications précédentes, **caractérisée par le fait qu'**elle présente un pH compris entre 3 et 12.

18. Procédé de teinture des fibres kératiniques et en particulier des fibres kératiniques humaines telles que les cheveux **caractérisé par le fait que** l'on applique sur ces fibres au moins une composition tinctoriale telle que définie à l'une quelconque des revendications 1 à 17 et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

19. Procédé selon la revendication 18, **caractérisé par le fait que** l'agent oxydant est choisi parmi le peroxyde d'hydrogène, le peroxyde d'urée, les bromates de métaux alcalins, les persels tels que les perborates et persulfates.

20. Dispositif à plusieurs compartiments, ou "kit" de teinture à plusieurs compartiments, dont un premier compartiment renferme une composition tinctoriale telle que définie à l'une quelconque des revendications 1 à 17 et un second compartiment renferme une composition oxydante.

## Patentansprüche

1. Zusammensetzung zum oxidativen Färben von Keratinfasern und insbesondere menschlichen Keratinfasern, wie dem Haar, **dadurch gekennzeichnet, daß** sie in einem zum Färben geeigneten Medium enthält:
- mindestens eine Oxidationsbase, die unter den p-Phenylendiaminen und/oder Bisphenylalkylendiaminen ausgewählt ist,
- mindestens ein m-Aminophenol als ersten Kuppler, und
- mindestens einen zweiten Kuppler, der unter 4-Hydroxyindolin und seinen Additionssalzen mit einer Säure ausgewählt ist.

2. Zusammensetzung nach Anspruch 1, **dadurch gekennzeichnet, daß** das m-Aminophenol oder die m-Aminophenole, die als erste Kuppler in den erfindungsgemäßen Zusammensetzungen verwendet werden, unter den Verbindungen, die der folgenden Formel (I) entsprechen, oder den Additionssalzen dieser Verbindungen mit einer Säure ausgewählt sind: 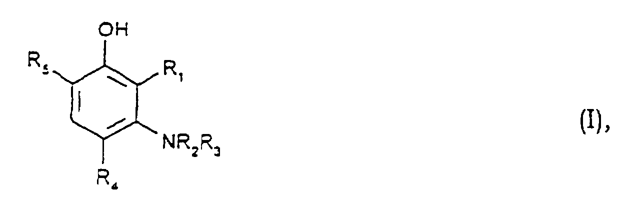 worin bedeuten:
- R₁ und R₄, die identisch oder voneinander verschieden sind, Wasserstoff, Halogen, C₁₋₄-Alkyl oder C₁₋₄-Alkoxy;
- R₂ Wasserstoff, C₁₋₄-Alkyl, C₁₋₄-Monohydroxyalkyl, C₂₋₄-Polyhydroxyalkyl, C₁₋₄-Trifluoralkyl oder Carbamoylmethyl;
- R₃ Wasserstoff oder C₁₋₄-Alkyl oder R₃ bildet mit R₂ und dem Stickstoffatom einen 5- oder 6-gliedrigen Heterocyclus;
- R₅ Wasserstoff, Halogen, C₁₋₄-Alkyl, C₁₋₄-Alkoxy, C₁₋₄-Monohydroxyalkyl oder C₂₋₄-Polyhydroxyalkyl.

3. Zusammensetzung nach Anspruch 1, **dadurch gekennzeichnet, daß** das m-Aminophenol oder die m-Aminophenole, die als erste Kuppler verwendet werden, unter 3-Amino-phenol, 5-Amino-2-methoxyphenol, 5-Amino-4-chlor-2-methyl-phenol, 5-Amino-2,4-dimethoxyphenol, 5-Amino-2-β-hydroxyethyloxy-phenol, 5-N-(β-Hydroxyethyl)amino-4-methoxy-2-methyl-phenol, 5-Amino-4-methoxy-2-methyl-phenol, 5-N-(γ-Hydroxypropyl)amino-2-methylphenol, 5-N-(β-Hydroxyethyl)amino-2-methyl-phenol, 5-Amino-2-methyl-phenol, 3-N-(Carbamoylmethyl)amino-phenol, 5-N-(Carbamoylmethyl)amino-2-methyl-phenol, 3-N,N-(Dimethyl)aminophenol, 3-N,N-(Diethyl)amino-phenol, 3-Amino-2,4-dichlor-phenol, 3-Amino-4,6-dichlor-phenoh, 5-Amino-6-chlor-2-methyl-phenol, 2-Chlor-5-N-(2',2',2'-trifluorethyl)amino-phenol, 5-Amino-4-chlor-2-methyl-phenol, 3-N-(Cyclopentyl)amino-phenol und den Additionssalzen dieser Verbindungen mit einer Säure ausgewählt sind.

4. Zusammensetzung nach einem der vorhergehenden Ansprüche, **dadurch gekennzeichnet, daß** die p-Phenylendiamine unter den Verbindungen der folgenden Formel (II) und deren Additionssalzen mit einer Säure ausgewählt sind: 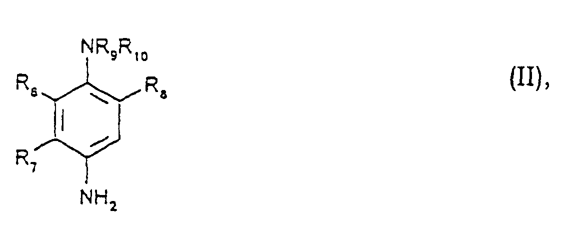 worin bedeuten:
- R₆, R₇ und R₈, die identisch oder voneinander verschieden sind, Wasserstoff, Halogen, C₁₋₄-Alkyl, C₁₋₄-Alkoxy, Sulfo, Carboxy, C₁₋₄-Monohydroxyalkyl oder C₂₋₄-Polyhydroxyalkyl,
- R₉ und R₁₀, die identisch oder voneinander verschieden sind, Wasserstoff, C₁₋₄-Alkyl, C₁₋₄-Monohydroxyalkyl, C₂₋₄-Polyhydroxyalkyl, C₁₋₄-Alkoxy-C₁₋₄-alkyl, C₁₋₄-Carbamoylalkyl, C₁₋₄-Mesylaminoalkyl, C₁₋₄-Acetylaminoalkyl, C₁₋₄-Ureidoalkyl, C₁₋₄-Carbalkoxy-C₁₋₄-aminoalkyl, C₁₋₄-Sulfoalkyl, C₁₋₄-Piperidinoalkyl, C₁₋₄-Morpholinoalkyl, Phenyl oder eine Phenylgruppe, die in p-Stellung mit einer Aminogruppe substituiert ist, oder R₉ und R₁₀ bilden mit dem Stickstoffatom, an das sie gebunden sind, einen Piperidino- oder Morpholino-Heterocyclus;
- mit der Maßgabe, daß mindestens eine der Gruppen R₆ oder R₈ Wasserstoff bedeuten muß, wenn R₉ und R₁₀ nicht gleichzeitig Wasserstoff bedeuten.

5. Zusammensetzung nach Anspruch 4, **dadurch gekennzeichnet, daß** die p-Phenylendiamine der Formel (II) ausgewählt sind unter: p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, 2-Methyl-5-methoxy-p-phenylendiamin, 2,6-Dimethyl-5-methoxy-p-phenylendiamin, N,N-Dimethyl-p-phenylendiamin, N,N-Diethyl-p-phenylendiamin, N,N-Dipropyl-p-phenylendiamin, 4-Amino-N,N-diethyl-3-methyl-anilin, N,N-Bis(β-hydroxyethyl)-p-phenylendiamin, 4-Amino-N,N-bis(β-hydroxyethyl)-3-methyl-anilin, 4-Amino-3-chlor-N,N-bis(β-hydroxyethyl)-anilin, 4-Amino-N,N-(ethyl, carbamoylmethyl)-anilin, 4-Amino-3-methyl-N,N-(ethyl, carbamoylmethyl)-anilin, 4-Amino-N,N-(ethyl, β-piperidinoethyl)-anilin, 4-Amino-3-methyl-N,N-(ethyl, β-piperidinoethyl)-anilin, 4-Amino-N,N-(ethyl, β-morpholinoethyl)-anilin, 4-Amino-3-methyl-N,N-(ethyl, β-morpholinoethyl)-anilin, 4-Amino-N,N-(ethyl, β-acetylaminoethyl)-anilin, 4-Amino-N-(β-methoxyethyl)-anilin, 4-Amino-3-methyl-N,N-(ethyl, β-acetylaminoethyl)-anilin, 4-Amino-N,N-(ethyl, β-mesylaminoethyl)-anilin, 4-Amino-3-methyl-N,N-(ethyl, β-mesylaminoethyl)-anilin, 4-Amino-N,N-(ethyl, β-sulfoethyl)-anilin, 4-Amino-3-methyl-N,N-(ethyl, β-sulfoethyl)-anilin, N-[(4'-Amino)phenyl]-morpholin, N-[(4'-Amino)phenyl]-piperidin, 2-β-Hydroxyethyl-p-phenylendiamin, 2-Fluor-p-phenylendiamin, 2-Carboxy-p-phenylendiamin, 2-Sulfo-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl, β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-β-Hydroxyethyloxy-p-phenylendiamin und deren Additionssalzen mit einer Säure.

6. Zusammensetzung nach Anspruch 5, **dadurch gekennzeichnet, daß** die p-Phenylendiamine der Formel (II) ausgewählt sind unter: p-Phenylendiamin, p-Toluylendiamin, 2,6-Dimethyl-p-phenylendiamin, 2-β-Hydroxyethyl-p-phenylendiamin, 2-Isopropyl-p-phenylendiamin, N-(β-Hydroxypropyl)-p-phenylendiamin, N,N-Bis(β-hydroxyethyl)-p-phenylendiamin, 4-Amino-N-(β-methoxyethyl)-anilin und deren Additionssalze mit einer Säure.

7. Zusammensetzung nach einem der vorhergehenden Ansprüche, **dadurch gekennzeichnet, daß** die Bisphenylalkylendiamine unter den Verbindungen der folgenden Formel (III) und deren Additionssalzen mit einer Säure ausgewählt sind: 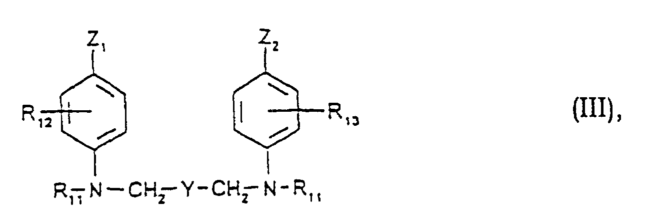 worin bedeuten:
- Z₁ und Z₂, die identisch oder voneinander verschieden sind, Hydroxy oder NHR₁₄, wobei R₁₄ Wasserstoff oder C₁₋₄-Alkyl bedeutet,
- R₁₁ Wasserstoff, C₁₋₄-Alkyl, C₁₋₄-Monohydroxyalkyl, C₂₋₄-Polyhydroxyalkyl oder C₁₋₄-Aminoalkyl, wobei die Aminogruppe substituiert sein kann,
- R₁₂ und R₁₃, die identisch oder voneinander verschieden sind, Wasserstoff, Halogen oder C₁₋₄-Alkyl,
- Y eine Gruppe, die unter den folgenden Gruppen ausgewählt ist:
-(CH₂)ₙ-; - (CH₂)ₘ-O-(CH₂)ₘ-; -(CH₂)ₘ-CHOH-(CH₂)ₘ- und 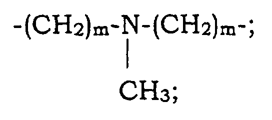 worin n Null oder eine ganze Zahl von 1 bis 8 und m Null oder eine ganze Zahl im Bereich von 1 bis 4 bedeutet.

8. Zusammensetzung nach Anspruch 7, **dadurch gekennzeichnet, daß** die Bisphenylalkylendiamine der Formel (III) unter N,N'-Bis(β-hydroxyethyl)-N,N'-bis(4'-aminophenyl)-1,3-diaminopropanol, N,N'-Bis(β-hydroxyethyl)-N,N'-bis(4'-aminophenyl)-ethylendiamin, N,N'-Bis(4-aminophenyl)-tetramethylendiamin, N,N'-Bis(β-hydroxyethyl)-N,N'-bis(4-aminophenyl)-tetramethylendiamin, N,N'-Bis(4-methylaminophenyl)-tetramethylendiamin, N,N'-Bis(ethyl)-N,N'-bis-(4'-amino, 3'-methylphenyl)-ethylendiamin und deren Additionssalzen mit einer Säure ausgewählt sind.

9. Zusammensetzung nach einem der vorhergehenden Ansprüche, **dadurch gekennzeichnet, daß** die Additionssalze mit einer Säure unter den Hydrochloriden, Hydrobromiden, Sulfaten und Tartraten ausgewählt sind.

10. Zusammensetzung nach einem der vorhergehenden Ansprüche, **dadurch gekennzeichnet, daß** das p-Phenylendiamin oder die p-Phenylendiamine und/oder das Bisphenylalkylendiamin oder die Bisphenylalkylendiamine 0,0005 bis 12 Gew.-% des Gesamtgewichts der Farbmittelzusammensetzung ausmachen.

11. Zusammensetzung nach Anspruch 10, **dadurch gekennzeichnet, daß** das p-Phenylendiamin oder die p-Phenylendiamine und/oder das Bisphenylalkylendiamin oder die Bisphenylalkylendiamine 0,005 bis 6 Gew.-% des Gesamtgewichts der Farbmittelzusammensetzung ausmachen.

12. Zusammensetzung nach einem der vorhergehenden Ansprüche, **dadurch gekennzeichnet, daß** das m-Aminophenol oder die m-Aminophenole der Formel (I) 0,0001 bis 10 Gew.-% des Gesamtgewichts der Farbmittelzusammensetzung ausmachen.

13. Zusammensetzung nach Anspruch 12, **dadurch gekennzeichnet, daß** das m-Aminophenol oder die m-Aminophenole der Formel (I) 0,005 bis 5 Gew.-% des Gesamtgewichts der Farbmittelzusammensetzung ausmachen.

14. Zusammensetzung nach einem der vorhergehenden Ansprüche, **dadurch gekennzeichnet, daß** das 4-Hydroxyindolin und/oder seine Additionssalze mit einer Säure 0,0001 bis 10 Gew.-% des Gesamtgewichts der Farbmittelzusammensetzung ausmachen.

15. Zusammensetzung nach Anspruch 14, **dadurch gekennzeichnet, daß** das 4-Hydroxyindolin und/oder seine Additionssalze mit einer Säure 0,005 bis 5 Gew.-% des Gesamtgewichts der Farbmittelzusammensetzung ausmachen.

16. Zusammensetzung nach einem der vorhergehenden Ansprüche, **dadurch gekennzeichnet, daß** das zum Färben geeignete Medium (oder der Träger) aus Wasser oder einem Gemisch von Wasser und mindestens einem organischen Lösungsmittel besteht, das unter niederen Alkanolen mit 1 bis 4 Kohlenstoffatomen, Glycerin, Glykolen, Glykolethern, aromatischen Alkoholen und deren Gemischen ausgewählt ist.

17. Zusammensetzung nach einem der vorhergehenden Ansprüche, **dadurch gekennzeichnet, daß** sie einen pH-Wert von 3 bis 12 besitzt.

18. Verfahren zum Färben von Keratinfasern und insbesondere menschlichen Keratinfasern, wie dem Haar, **dadurch gekennzeichnet, daß** auf die Fasern mindestens eine Farbmittelzusammensetzung nach einem der Ansprüche 1 bis 17 aufgebracht wird und die Farbe bei einem sauren, neutralen oder alkalischen pH-Wert mit einem Oxidationsmittel entwickelt wird, das unmittelbar bei der Anwendung zu der Farbmittelzusammensetzung gegeben wird oder das in einer oxidierenden Zusammensetzung vorliegt, die gleichzeitig oder getrennt davon anschließend aufgebracht wird.

19. Verfahren nach Anspruch 18, **dadurch gekennzeichnet, daß** das Oxidationsmittel unter Wasserstoffperoxid, Harnstoffperoxid, Alkalimetallbromaten und Salzen von Persäuren, wie Perboraten und Persulfaten, ausgewählt ist.

20. Vorrichtung mit mehreren Abteilungen oder "Kit" zum Färben mit mehreren Abteilungen, wobei eine erste Abteilung eine Farbmittelzusammensetzung nach einem der Ansprüche 1 bis 17 und eine zweite Abteilung eine oxidierende Zusammensetzung enthält.

## Claims

1. Composition for the oxidation dyeing of keratin fibres, and in particular human keratin fibres such as the hair, **characterized in that** it comprises, in a medium which is suitable for dyeing:
- at least one oxidation base chosen from para-phenylenediamines and/or bis(phenyl)alkylenediamines,
- at least one meta-aminophenol as first coupler,
- at least one second coupler chosen from 4-hydroxyindoline and the addition salts thereof with an acid.

2. Composition according to Claim 1, **characterized in that** the meta-aminophenol(s) which are used as first coupler is (are) chosen from compounds corresponding to formula (I) below, and the addition salts thereof with an acid: 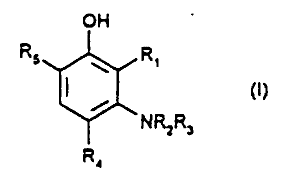 in which:
R₁ and R₄, which may be identical or different, represent a hydrogen or halogen atom or a C₁-C₄ alkyl or C₁-C₄ alkoxy radical,
R₂ represents a hydrogen atom or a C₁-C₄ alkyl, C₁-C₄ monohydroxyalkyl, C₂-C₄ polyhydroxyalkyl, C₁-C₄ trifluoroalkyl or carbamoylmethyl radical,
R₃ represents a hydrogen atom or a C₁-C₄ alkyl radical or alternatively forms, with R₂ and the nitrogen atom, a 5- or 6-membered heterocycle,
R₅ represents a hydrogen or halogen atom or a C₁-C₄ alkyl, C₁-C₄ alkoxy, C₁-C₄ monohydroxyalkyl or C₂-C₄ polyhydroxyalkyl radical.

3. Composition according to Claim 1, **characterized in that** the meta-aminophenol(s) used as first coupler is (are) chosen from 3-aminophenol, 5-amino-2-methoxyphenol, 5-amino-4-chloro-2-methyl-phenol, 5-amino-2,4-dimethoxyphenol, 5-amino-2-β-hydroxyethyloxyphenol, 5-N-(β-hydroxyethyl)amino-4-methoxy-2-methylphenol, 5-amino-4-methoxy-2-methylphenol, 5-N-(γ-hydroxypropyl)amino-2-methylphenol, 5-N-(β-hydroxyethyl)amino-2-methyl-phenol, 5-amino-2-methylphenol, 3-N-(carbamoylmethyl)-aminophenol, 5-N-(carbamoylmethyl)amino-2-methylphenol, 3-N,N-(dimethyl)aminophenol, 3-N,N-(diethyl)aminophenol, 3-amino-2,4-dichlorophenol, 3-amino4,6-dichlorophenol, 5-amino-6-chloro-2-methylphenol, 2-chloro-5-N-(2',2',2'-trifluoroethyl)-aminophenol, 5-amino-4-chloro-2-methylphenol and 3-N-(cyclopentyl)-aminophenol, and the addition salts thereof with an acid.

4. Composition according to any one of the preceding claims, **characterized in that** the para-phenylenediamines are chosen from compounds of formula (II) below, and the addition salts thereof with an acid: 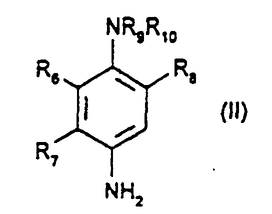 in which:
R₆, R₇ and R₈, which may be identical or different, represent a hydrogen or halogen atom or a C₁-C₄ alkyl, C₁-C₄ alkoxy, sulpho, carboxyl, C₁-C₄ monohydroxyalkyl or C₂-C₄ polyhydroxyalkyl radical,
R₉ and R₁₀, which may be identical or different, represent a hydrogen atom, a C₁-C₄ alkyl, C₁-C₄ monohydroxyalkyl, C₂-C₄ polyhydroxyalkyl, C₁-C₄ alkoxy(C₁-C₄)alkyl, carbamyl(C₁-C₄)alkyl, C₁-C₄ mesylaminoalkyl, acetylamino(C₁-C₄)alkyl, C₁-C₄ ureidoalkyl, carb(C₁-C₄)alkoxyamino(C₁-C₄)alkyl, C₁-C₄ sulphoalkyl, piperidino(C₁-C₄)alkyl, morpholino(C₁-C₄)alkyl or phenyl radical or a phenyl radical substituted in the para position with an amino group, or alternatively R₉ and R₁₀ form, together with the nitrogen atom to which they are attached, a piperidino or morpholino heterocycle;
it being understood that if R₉ and R₁₀ do not simultaneously represent a hydrogen atom, then at least one of the radicals R₆ and R₈ must represent a hydrogen atom.

5. Composition according to Claim 4, **characterized in that** the para-phenylenediamines of formula (II) are chosen from para-phenylenediamine, paratoluylenediamine, 2-chloro-para-phenylenediamine, 2,3-dimethyl-para-phenylenediamine, 2,6-dimethylpara-phenylenediamine, 2,6-diethyl-para-phenylenediamine, 2,5-dimethyl-para-phenylenediamine, 2-methyl-5-methoxy-para-phenylenediamine, 2,6-dimethyl-5-methoxy-para-phenylenediamine, N,N-dimethyl-para-phenylenediamine, N,N-diethyl-paraphenylenediamine, N,N-dipropyl-para-phenylenediamine, 4-amino-N,N-diethyl-3-methylaniline, N,N-bis(β-hydroxyethyl)-para-phenylenediamine, 4-amino-N,N-bis(β-hydroxyethyl)-3-methylaniline, 4-amino-3-chloro-N,N-big(β-hydroxyethyl)aniline, 4-amino-N,N-(ethyl, carbamylmethyl)aniline, 4-amino-3-methyl-N,N-(ethyl, carbamylmethyl)aniline, 4-amino-N,N-(ethyl, β-piperidinoethyl)aniline, 4-amino-3-methyl-N,N-(ethyl, β-piperidinoethyl)-aniline, 4-amino-N,N-(ethyl, β-morpholinoethyl)-aniline, 4-amino-3-methyl-N,N-(ethyl, β-morpholinoethyl)aniline, 4-amino-N,N-(ethyl, β-acetylaminoethyl)aniline, 4-amino-N-(β-methoxyethyl)-aniline, 4-amino-3-methyl-N,N-(ethyl, β-acetylamino-ethyl)aniline, 4-amino-N,N-(ethyl, β-mesylaminoethyl)-aniline, 4-amino-3-methyl-N,N-(ethyl, β-mesylamino-ethyl)aniline, 4-amino-N,N-(ethyl, β-sulphoethyl)-aniline, 4-amino-3-methyl-N,N-(ethyl, β-sulphoethyl)-aniline, N-[(4'-amino)phenyl]-morpholine, N-[(4'-amino)-phenyl]piperidine, 2-β-hydroxyethyl-para-phenylene-diamine, 2-fluoropara-phenylenediamine, 2-carboxy-para-phenylenediamine, 2-sulpho-para-phenylenediamine, 2-isopropyl-para-phenylenediamine, N-(β-hydroxypropyl)-para-phenylenediamine, 2-hydroxymethyl-paraphenylene-diamine, N,N-dimethyl-3-methyl-paraphenylenediamine, N,N-(ethyl, β-hydroxyethyl)-para-phenylenediamine, N-(β,γ-dihydroxypropyl)-para-phenylenediamine, N-(4'-aminophenyl)-paraphenylenediamine, N-phenyl-para-phenylenediamine and 2-β-hydroxyethyloxy-para-phenylenediamine, and the addition salts thereof with an acid.

6. Composition according to Claim 5, **characterized in that** the para-phenylenediamines of formula (II) are chosen from para-phenylenediamine, paratoluylenediamine, 2,6-dimethyl-para-phenylenediamine, 2-β-hydroxyethyl-para-phenylene-diamine; 2-isopropyl-para-phenylenediamine, N-(β-hydroxypropyl)-para-phenylenediamine, N,N-bis-(β-hydroxyethyl)-para-phenylenediamine and 4-amino-N-(β-methoxyethyl)aniline, and the addition salts thereof with an acid.

7. Composition according to any one of the preceding claims, **characterized in that** the bis(phenyl)-alkylenediamines are chosen from the compounds of formula (III) below, and the addition salts thereof with an acid: 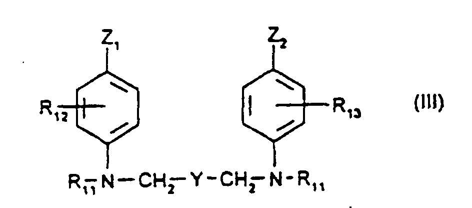 in which:
Z₁ and Z₂, which may be identical or different, represent a hydroxyl radical or a radical NHR₁₄ in which R₁₄ represents a hydrogen atom or a C₁-C₄ alkyl radical,
R₁₁ represents a hydrogen atom or a C₁-C₄ alkyl, C₁-C₄ monohydroxyalkyl or C₂-C₄ polyhydroxyalkyl radical or a C₁-C₄ aminoalkyl radical in which the amino residue may be substituted,
R₁₂ and R₁₃, which may be identical or different, represent a hydrogen or halogen atom or a C₁-C₄ alkyl radical,
Y represents a radical taken from the group consisting of the following radicals: - (CH₂)ₙ-; -(CH₂)ₘ-O- (CH₂)ₘ-; -(CH₂)ₘ-CHOH-(CH₂)ₘ- and 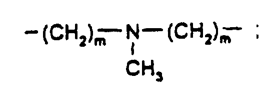 in which n is an integer between 0 and 8 inclusive and m is an integer between 0 and 4 inclusive.

8. Composition according to Claim 7, **characterized in that** the bis(phenyl)alkylenediamines of formula (III) are chosen from N,N'-bis(β-hydroxy-ethyl)-N,N'-bis(4'-aminophenyl)-1,3-diaminopropanol, N,N'-bis(β-hydroxyethyl)-N,N'-bis(4'-aminophenyl)-ethylenediamine, N,N'-bis(4-aminophenyl)tetra-methylenediamine, N,N'-bis-(β-hydroxyethyl)-N,N'-bis-(4-aminophenyl)tetramethylenediamine, N,N'-bis-(4-methylaminophenyl)-tetramethylenediamine and N,N'-bis(ethyl)-N,N'-bis(4'-amino-3'-methylphenyl)-ethylenediamine, and the addition salts thereof with an acid.

9. Composition according to any one of the preceding claims, **characterized in that** the addition salts with an acid are chosen from the hydrochlorides, hydrobromides, sulphates and tartrates.

10. Composition according to any one of the preceding claims, **characterized in that** the para-phenylenediamine(s) and/or the bis(phenyl)alkylenediamine(s) represent from 0.0005 to 12% by weight relative to the total weight of the dye composition.

11. Composition according to Claim 10, **characterized in that** the para-phenylenediamine(s) and/or the bis(phenyl)alkylenediamine(s) represent from 0.005 to 6% by weight relative to the total weight of the dye composition.

12. Composition according to any one of the preceding claims, **characterized in that** the metaaminophenol(s) of formula (I) represent from 0.0001 to 10% by weight relative to the total weight of the dye composition.

13. Composition according to Claim 12, **characterized in that** the meta-aminophenol(s) of formula (I) represent from 0.005 to 5% by weight relative to the total weight of the dye composition.

14. Composition according to any one of the preceding claims, **characterized in that** the 4-hydroxyindoline and/or the addition salt(s) thereof with an acid represent from 0.0001 to 10% by weight relative to the total weight of the dye composition.

15. Composition according to Claim 14, **characterized in that** the 4-hydroxyindoline and/or the addition salt(s) thereof with an acid represent from 0.005 to 5% by weight relative to the total weight of the dye composition.

16. Composition according to any one of the preceding claims, **characterized in that** the medium which is suitable for dyeing (or the support) consists of water or a mixture of water and at least one organic solvent chosen from C₁-C₄ lower alkanols, glycerol, glycols and glycol ethers, aromatic alcohols and mixtures thereof.

17. Composition according to any one of the preceding claims, **characterized in that** it has a pH of between 3 and 12.

18. Process for dyeing keratin fibres, and in particular human keratin fibres such as the hair, **characterized in that** at least one dye composition as defined in any one of Claims 1 to 17 is applied to these fibres and the colour is developed at acidic, neutral or alkaline pH using an oxidizing agent which is added to the dye composition only at the time of use, or which is present in an oxidizing composition that is applied simultaneously or sequentially in a separate manner.

19. Process according to Claim 18, **characterized in that** the oxidizing agent is chosen from hydrogen peroxide, urea peroxide, alkali metal bromates and persalts such as perborates and persulphates.

20. Multi-compartment device or multi-compartment dyeing "kit", a first compartment of which contains a dye composition as defined in any one of Claims 1 to 17 and a second compartment of which contains an oxidizing composition.
